# EUROPEAN PATENT APPLICATION

(11) **EP 2 283 794 A1**
(43) Date of publication of application: **16.02.2011**
(21) Application number: 09466026.3
(22) Date of filing: 16.12.2009
(51) Int. Cl.: A61F 2/90

(54) **Biodegradable stent**

(30) Priority: 11.08.2009 CZ 200921563 U
(71) Applicant: Ella-CS, s.r.o., 506 06 Hradec Králové (CZ)
(72) Inventor: Petr, Kubena Petr, 500 09 Hradec Králové (CZ); Semiková, Barbora, 503 02 Predmerice nad Labem (CZ)

(57) **Abstract**

The surface of the biodegradable stent is made as complex fine mesh in shape of rounded ( 2.1 ) rings ( 2 ) and the stent is tubular or conical. Favourably the stent will be made of degradable polymer thread and can be covered externally by the biodegradable covering.

## Description

### Technical scone

Biodegradable stents are designed especially to be placed into oesophagus to stop the variceal bleeding. It is also possible to place it in other tubular organs.

### Existing technical background

Biodegradable stents used to stop oesophageal bleeding according to the EP 1 795 151 are well known. The issue of stent migration is resolved in the same way as it is with metallic stents - by broadening their ends. In case of EP 1 795 151 there is an anti-migration means even under these broadened ends in form of fibre with an open end. This solution positively affects migration but the broadened ends put too much pressure on the tissue. In other stents that are not made of biodegradable thread and are to be removed after some time in the body there are different anti-migration collars, protrusions or joint wire coils. When the biodegradable thread is used these mechanisms cannot be used; moreover these stents bend at point of the joint coils which is an undesirable effect. Therefore the production of biodegradable stent to be used in oesophagus with other anti-migration means than flared ends has not been produced yet.

The aim of this technical solution is to create such a design of biodegradable stent that will not be flared at both ends and will not rnigrate.

### The essence of technical solution

The target is possible to reach by biodegradable stent with integrated mesh in form of rounded rings. After the implantation the stent is pressed into the tissue which precludes migration. The technical solution enables to braid such a mesh from biodegradable thread and at the same time secures its anti-migration character. Favourably the stent will be made of degradable polymer thread and can be covered externally by the biodegradable covering.

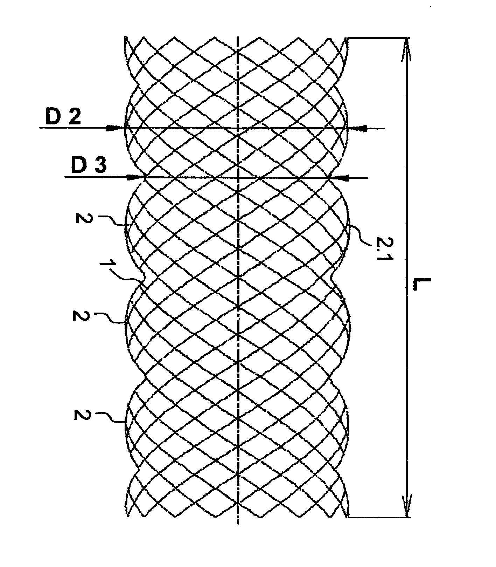

### The drawing

The attached technical drawing shows the described shape in detail.

### An example of technical solution

This particular case concerns biodegradable stent made of absorbable polydioxanone thread 1 and is coiled in a mesh. The surface is shaped tubular or conically in rounded 2.1 rings 2 with the broadest diameter of rounding D2 = 30 mm and the narrowest diameter of rounding D3 = 26 mm. The length of this stent L = 80 mm. The stent is self-expandable and is thermally processed by heating to minimally 80° C. The stent is braided from monofilament thread but can simultaneously be braided from double thread.

During the implantation the biodegradable stent is led to the desired place of the tubular organ by means of delivery system. In the system the stent is longitudinally comprised and after the deployment it relaxes and expands radialy. The delivery system is then taken out of the patient. After some time the structure of the biodegradable thread is disturbed and it degrades by hydrolysis. End products are water and carbon dioxide. The degradation results in decomposition of the stent to small debris and their absorption or leaving the body by natural ways.

## Claims

1. The biodegradable stent **characterized in that** its outer surface is made from biodegradable material as integrated mesh in shape of consecutive rounded (2.1) rings (2).

2. The biodegradable stent as described in the claim 1 **characterized in that** it is covered with external biodegradable covering.

3. The biodegradable stent as described in the clims 1 or 2 **characterized in that** the biodegradable material is degradable polymer thread.
